Europäisches Patentamt

**European Patent Office**

Office européen des brevets

(19)

(11) Veröffentlichungsnummer: **0 039 788**
B1

(12) # EUROPÄISCHE PATENTSCHRIFT

(45) Veröffentlichungstag der Patentschrift:
28.09.83

(21) Anmeldenummer: 81102722.6

(22) Anmeldetag: 10.04.81

(51) Int. Cl.³: **A 01 N 37/02,** A 01 N 37/04,
A 01 N 37/06, C 07 C 53/126,
C 07 C 55/02, C 07 C 57/12,
C 08 F 8/44

(54) **Kupferkomplexe, ihre Herstellung, ihre Verwendung zur Bekämpfung von Pflanzenschädlingen und Mittel dafür.**

(30) Priorität: 03.05.80 DE 3017123
14.06.80 DE 3022432
18.10.80 DE 3039409

(43) Veröffentlichungstag der Anmeldung:
18.11.81 Patentblatt 81/46

(45) Bekanntmachung des Hinweises auf die Patenterteilung:
28.09.83 Patentblatt 83/39

(84) Benannte Vertragsstaaten:
AT BE CH DE FR IT LI NL SE

(56) Entgegenhaltungen:
DE-A-2 202 448
DE-A-2 807 293
FR-A-970 321
FR-A-1 590 623
GB-A-593 416
GB-A-599 443
GB-A-1 394 990
US-A-4 020 180
US-A-4 193 993

(73) Patentinhaber: **BASF Aktiengesellschaft,**
**Carl-Bosch-Strasse 38, D-6700 Ludwigshafen (DE)**

(72) Erfinder: **Kraft, Helmut, Hirtenweg 131,**
**D-6719 Wattenheim (DE)**
Erfinder: **Schumacher, Heinz, Karlsruher Strasse 19,**
**D-6940 Weinheim (DE)**
Erfinder: **Pommer, Ernst-Heinrich, Dr., Berliner Platz 7,**
**D-6703 Limburgerhof (DE)**
Erfinder: **Schlotterbeck, Dietrich, Dr., Mainzer**
**Strasse 38, D-6703 Limburgerhof (DE)**
Erfinder: **Ley, Gregor, Dr., In den Trankstuecken,**
**D-6719 Wattenheim (DE)**

EP 0 039 788 B1

Kupferkomplexe, ihre Herstellung, ihre Verwendung zur Bekämpfung von
Pflanzenschädlingen und Mittel dafür

Kupfersalze werden bereits seit langem in der Landwirtschaft zur Bekämpfung von Pilzkrankheiten an Kulturpflanzen eingesetzt. Um die Wirksamkeit der Kupferbehandlung von Kulturen über einen längeren Zeitraum zu garantieren, werden meist in Wasser schwer oder unlösliche anorganische Kupfersalze verwendet, z. B. Kupferoxichlorid. In letzter Zeit sind auch ölige Formulierungen von Kupfersalzen auf der Basis niedermolekularer organischer Carbonsäuren bekannt geworden (vgl. Technisches Bulletin der Firma Complex Quimica S. A. über Complex-200), die bei gleicher Wirksamkeit einen erheblich niedrigeren Kupfergehalt als die herkömmlichen Produkte haben.

Weiter sind bereits in der GB-PS 1 394 990 wasserlösliche Copolymere angegeben, die durch Polymerisation von 20 bis 60 Teilen Acrylsäure oder Methacrylsäure, 20 bis 70 Teilen Alkylacrylat oder -methacrylat und 5 bis 20 Teilen eines weichmachenden Monomeren entstehen sollen und in die Pflanzenschutzmittel eingelagert werden sollen. Die sauren Copolymere sollen dabei auch in Form ihrer Kupfersalze mit dem Pflanzenschutzmittel kombiniert werden können. Bei eigenen Versuchen, diese GB-PS nachzuarbeiten, wurden jedoch keine brauchbaren Produkte erhalten.

Schließlich sind aus der DE-OS 2 807 293 und der US-PS 3 900 504 Kupferamminkomplexe mit fungizider Wirkung bekannt, die sich jedoch nicht zum Besprühen von Pflanzen eignen, da sie nicht auf diesen haften.

Die GB-PS 599 443 beschreibt Kupferamminkomplexe nicht polymerer Carbonsäuren und deren Anwendung auf dem Textilgebiet. Gemäß der GB-PS 593 416 kann man Gegenstände — wie Textilien, Holz usw. — mit einem Kupferammin-naphtat überziehen, um sie gegen Fungi-Befall zu schützen. Über die Brauchbarkeit der Kupferkomplexe im Pflanzenschutz wird in den beiden PS nichts ausgesagt.

In der DE-OS 2 202 448 werden Holzschutzmittel beschrieben. Es wird nichts über eine Anwendung in der Landwirtschaft ausgesagt.

Gemäß der US-PS 4 193 993 kann z. B. eine Dipentenmono- oder -dicarbonsäure in Form ihres Ammin-Komplexes als Fungizid verwendet werden.

Es wurden nun einfache pflanzenverträgliche Kupferamminsalze von Polycarbonsäuren gefunden, die als Säurebestandteil 60—100% Acrylsäure und/oder Methacrylsäure und 0—40% Acrylsäure- oder Methacrylsäureester enthalten und gute fungizide Eigenschaften, vorzugsweise für den Pflanzenschutz, haben.

Gegenstand der Erfindung sind ferner Pestizide, die das pflanzenverträgliche Kupferamminsalz enthalten.

Das Kupfer liegt als Amminkomplex vor, wobei das Ammoniak in dem Komplex zum Teil auch durch niedermolekulare, leicht flüchtige Amine,
wie Methylamin, ersetzt sein kann.

Als Anionen haben sich die von Acrylsäure und Methacrylsäure und ihren Copolymeren mit Acrylsäureestern und Methacrylsäureestern oder Mischungen daraus als gut geeignet erwiesen.

Als besonders günstig haben sich Mischungen aus wäßrigen Lösungen eines Kupferammin-Salzes und des Ammoniumsalzes eines Polymeren erwiesen, welches aus 60 bis 100% Acrylsäure oder Methacrylsäure und 0 bis 40% Acrylsäure- oder Methacrylsäureester besteht.

Es ist nicht erforderlich, die Kupferamminsalze in reiner Form als Pflanzenschutzmittel zu verwenden.

Das erfindungsgemäße Pflanzenschutzmittel enthält das Kupfer vorzugsweise in einer Menge von 0,01 bis 10%.

Die Herstellung der erfindungsgemäßen Kupfersalze erfolgt am einfachsten durch Zugabe von Ammoniak oder Amin zu einem leicht löslichen Kupfersalz, wie Kupfer(II)-sulfat, und anschließenden Zusatz der Carbonsäure. Sie kann auch durch Umsetzung von Kupfersalzen schwacher oder leicht flüchtiger Säuren mit den Polycarbonsäuren, die gegebenenfalls teilweise mit Ammoniak neutralisiert sein können, und anschließende Umsetzung mit Ammoniak bzw. Ammoniakwasser erfolgen. Die Konzentrationen der Reaktionspartner wird dabei in annähernd molarem Verhältnis eingestellt. Ein größerer Überschuß an Säure ist ungünstig; ein Überschuß an Ammoniak oder Amin stört nicht, ein Unterschuß führt jedoch zu Fällungen, insbesondere beim Verdünnen mit Wasser auf anwendungsgerechte Konzentrationen.

Die Polymeren können nach den üblichen Verfahren der Substanz-, Lösungs-, Fällungs-, Emulsions- oder Suspensionspolymerisation erhalten werden.

So kann man beispielsweise die Monomeren unter Ausschluß von Sauerstoff in Isopropanol/Wasser 1:1 lösen, so daß eine 10 bis 50%ige Lösung entsteht. Hierzu gibt man etwas 0,5 bis 5% (bezogen auf die Menge der Monomeren) Polymerisationsinitiator, z. B. Azobisisobutyronitril, und erwärmt die Mischung 5 Stunden auf 80° C. Die so erhaltenen Polymeren werden direkt oder nach Isolierung zur Herstellung des erfindungsgemäßen Pflanzenschutzmittels verwendet. Die folgenden Beispiele sollen die Erfindung erläutern. Alle Mengen- und Prozentangaben beziehen sich auf Gewichte.

Beispiel 1

Zu einer Lösung eines Lösungscopolymeren mit einem K-Wert von K=46 aus 6,7 Teilen Methylacrylat und 13,3 Teilen Acrylsäure in 96,25 Teilen Wasser und 3,75 Teilen konzentriertem Ammoniak wird unter Rühren eine Lösung von 30

Teilen CuSO$_4$ · 5 H$_2$O in 75 Teilen Wasser und 45 Teilen konzentriertem Ammoniak gegeben. Die erhaltene tiefblaue, klare Lösung ist lagerstabil und mit Wasser in jedem Verhältnis mischbar. Der Kupfergehalt der Lösung beträgt 2,87%.

### Beispiel 2

Es wird wie in Beispiel 1 verfahren, jedoch wird ein Lösungscopolymerisat mit einem K-Wert von 42 aus 6,7 Teile Ethylacrylat und 13,3 Teilen Acrylsäure verwendet.

### Beispiel 3

Es wird wie in Beispiel 1 verfahren, jedoch wird ein Lösungscopolymerisat vom K-Wert 41 aus 6,7 Teilen n-Butylacrylat und 13,3 Teilen Acrylsäure verwendet.

### Beispiel 4

Zu einer 50%igen Lösung eines Copolymeren des K-Wertes K=48 aus 25,1 Teilen Acrylsäure und 12,4 Teilen n-Butylacrylat in Isopropanol/Wasser (1 : 1) werden 37,5 Teile Wasser und 7,5 Teile konzentriertes Ammoniak gegeben. Dann werden 60 Teile CuSO$_4$ · 5 H$_2$O in 50 Teilen Wasser in der Hitze gelöst und zu dieser Lösung nach Abkühlen 120 Teile konzentriertes Ammoniak gegeben. Die erhaltenen Lösungen werden vereinigt. Es entsteht eine tiefblaue, klare Lösung, die lagerstabil und mit Wasser in jedem Verhältnis mischbar ist. Der Kupfergehalt der Lösung beträgt 4,36%.

### Beispiel 5

Zu einer 25%igen Lösung eines Copolymeren des K-Werts K=43 aus 12,5 Teilen Acrylsäure und 6,2 Teilen n-Butylacrylat in Isopropanol/Wasser (1 : 1) werden 37,5 Teile Wasser und 3,75 Teile konzentriertes Ammoniak gegeben. 20 Teile CuSO$_4$ · 5 H$_2$O werden in 75 Teilen Wasser heiß gelöst. Die Lösung wird abgekühlt und mit 35 Teilen konzentriertem Ammoniak versetzt. Die erhaltenen Lösungen werden vereinigt. Der Kupfergehalt der Lösung beträgt 2,11%.

### Beispiel 6

Zu der Lösung eines Lösungspolymerisats mit einem K-Wert von K=20 aus 4,5 Teilen Acrylsäure in 33 Teilen Wasser und 10 Teilen konzentriertem Ammoniak wird eine Lösung aus 30 Teilen CuSO$_4$ · 5 H$_2$O in 60 Teilen konzentriertem Ammoniak gegeben. Die erhaltene tiefblaue Lösung ist lagerstabil und mit Wasser in jedem Verhältnis mischbar. Der Kupfergehalt der Lösung beträgt 5,53%.

### Beispiel 7

Zu einer Lösung von 45 Teilen CuSO$_4$ · 5 H$_2$O in 90 Teilen konzentriertem Ammoniak wird die Lösung eines Lösungspolymerisats mit einem K-Wert von etwa K=20 aus 4,5 Teilen Acrylsäure in 49 Teilen Wasser und 10 Teilen konzentriertem Ammoniak zugetropft. Der Kupfergehalt der Lösung beträgt 5,78%.

### Beispiel 8

Zu einer Lösung von 15 Teilen CuSO$_4$ · H$_2$O in 10 Teilen Wasser und 40 Teilen konzentriertem Ammoniakwasser wurde langsam unter Rühren eine Mischung von 10 Teilen einer 25%igen Lösung von Polymethacrylsäure mit einem K-Wert von 60 in Propylenglykol und 10 Teilen Wasser zugetropft. Es entstand eine tiefblaue klare Lösung, die mit Wasser in jedem Verhältnis mischbar ist. Beim Auftrocknen der (verdünnten) Lösung bei Raumtemperatur entsteht eine in Wasser schwer lösliche Kupferverbindung.

### Beispiel 9

Zu einer Lösung aus 11 Teilen CuSO$_4$ · 5 H$_2$O in 15 Teilen Wasser und 25 Teilen konzentriertem Ammoniakwasser wurde eine Mischung von 38 Teilen einer 40%igen Dispersion auf Basis von 90 Gew.-% n-Butylacrylat und 10 Gew.-% Acrylsäure, hergestellt durch Emulsionspolymerisation, 40 Teilen Wasser und 3 Teilen konzentriertem Ammoniakwasser langsam zugetropft. Es entsteht eine blaue, milchig-trübe Dispersion, die mit Wasser in jedem Verhältnis mischbar ist und beim Auftrocknen bei Raumtemperatur einen wasserfesten Film ergibt.

### Beispiel 10

Zu einer Lösung von 45 Teilen CuSO$_4$ · 5 H$_2$O in 90 Teilen konzentriertem Ammoniakwasser wird eine Mischung aus 10 Teilen einer 50%igen Lösung einer Polyacrylsäure vom K-Wert 25, 10 Teilen konzentriertem Ammoniakwasser und 43 Teilen Wasser langsam zugetropft. Die entstandene tiefblaue, klare Lösung wird im Verhältnis 1 : 1 mit der Kunststoffdispersion® Acronal 567 D abgemischt. Die entstandene blaugefärbte, milchig-trübe Dispersionsmischung ist mit Wasser in jedem Verhältnis mischbar und gibt beim Auftrocknen einen wasserfesten Film.

### Beispiel 11

Zu einer Lösung aus 60 Teilen CuSO$_4$ · 5 H$_2$O in 50 Teilen Wasser und 120 Teilen konzentriertem Ammoniakwasser wird eine Lösung aus 75 Teilen einer 50%igen Lösung eines Copolymeren aus 33 Gew.-% n-Butylacrylat, 65 Gew.-% Acrylsäure und 2 Gew.-% Dimethylaminoethylmethacrylat, das mit Methylchlorid quaterniert wurde, vom K-Wert 48, 37,5 Teilen Wasser und 7,5 Teilen konzentriertem Ammoniakwasser unter Rühren zugegeben. Es entsteht eine tiefblaue lagerstabile, mit Wasser in allen Verhältnissen mischbare Lösung, die beim Auftrocknen einen wasserfesten Trockenrückstand ergibt.

### Beispiel 12

Zu einer Lösung aus 30 Teilen $CuSO_4 \cdot 5 H_2O$ in 75 Teilen Wasser und 45 Teilen konzentriertem Ammoniakwasser wurde eine Mischung aus 75 Teilen einer 25%igen Lösung eines Copolymeren aus 33 Gew.-% tert.-Butylacrylat und 67 Gewichtsteilen Acrylsäure vom K-Wert 45, 37,5 Gewichtsteilen Wasser und 3,75 Gewichtsteilen konzentriertem Ammoniakwasser unter Rühren zugegeben. Die entstandene tiefblaue Lösung ist lagerstabil, mit Wasser in jedem Verhältnis mischbar und ergibt beim Auftrocknen einen spröden, wasserfesten Trockenrückstand.

### Beispiel 13

Zu einer Lösung aus 40 Teilen $CuSO_4 \cdot 5 H_2O$ in 75 Teilen Wasser und 50 Teilen konzentriertem Ammoniakwasser wurde die klare Lösung aus 75 Teilen einer 25%igen Lösung eines Copolymeren aus 33 Gew.-% n-Butylacrylat und 67 Gew.-% Acrylsäure vom K-Wert 40, 10 Teilen Ölsäure, 37,5 Teilen Wasser und 25 Teilen konzentriertem Ammoniakwasser unter Rühren zugegeben. Die entstandene klare, tiefblaue Lösung ergibt beim Verdünnen mit Wasser eine ausreichend stabile trübe Spritzbrühe, die beim Auftrocknen einen kupferhaltigen Trockenrückstand ergibt, der mit Wasser zwar quillt, aber sich nicht auflöst.

### Beispiel 14

Zu einer Lösung aus 30 Teilen $CuSO_4 \cdot 5 H_2O$ in 75 Teilen Wasser und 45 Teilen Ammoniakwasser wurde unter Rühren eine Lösung aus 75 Teilen einer 25%igen Lösung eines Copolymeren aus 33 Gewichtsteilen Styrol und 67 Gewichtsteilen Acrylsäure vom K-Wert 36, 37,5 Teilen Wasser und 3,75 Teilen konzentriertem Ammoniakwasser zugegeben. Die tiefblaue Lösung ist lagerstabil und mit Wasser in jedem Verhältnis mischbar; nach dem Auftrocknen entsteht ein wasserfester Rückstand.

Trägt man das erfindungsgemäße auf Anwendungskonzentrationen verdünnte Pflanzenschutzmittel auf zu behandelnde Gegenstände, Pflanzen oder Pflanzenteile auf, so entsteht beim Auftrocknen der Lösung ein in Wasser schwer oder nicht lösliches Metallpolysalz, welches fest auf dem Gegenstand oder der Pflanze haftet und seine fungizide bzw. bakterizide Wirkung über einen langen Zeitraum behält.

Die neuen Komplexe besitzen beispielsweise eine ausgezeichnete fungizide Wirkung, welche die von bislang bekannten kupferhaltigen Fungiziden übertrifft. Sie sind daher überall dort einsetzbar, wo unerwünschter Bewuchs oder Befall durch Organismen auftritt. Beispiele hierfür sind die Hemmung von Bakterien-, Algen-, Pilz-, Flechten- und Moosbefall von Pflanzen sowie von Bauteilen wie Natur- und Kunststein, Pflaster und Fassaden, Putzen, Anstrichen, und dem Wasser ausgesetzten Holzteilen. Insbesondere eignen sich die neuen Komplexe zur Bekämpfung von Phytophthora infestans an Tomaten und Kartoffeln, Plasmopara viticola an Reben, Pseudoperonospora humuli an Hopfen, Cercospora beticola an Rüben, Cercospora musae an Bananen, Venturia inaequalis an Äpfeln, Exobasidium vexans an Tee, Hemileia vastatrix an Kaffee.

Überraschenderweise eignen sich die neuen Pflanzenschutzmittel auch vorzüglich als Bakterizide zur Bekämpfung von Pflanzenbakteriosen. Folgende Bakteriosen lassen sich beispielsweise mit den neuen Mitteln bekämpfen:

Corynebacterium michiganense an Tabak, Erwinia amylovora an Birnen und Äpfeln, Erwinia carotovora an Kartoffeln, Pseudomonas lachrymans an Gurken, Pseudomonas phaseolicola an Bohnen, Pseudomonas syringae an Flieder, Pseudomonas solanacearum an Bananen, Xanthomonas campestris an Kohl, Xanthomonas malvacearum an Baumwolle und Xanthomonas oryzae an Reis.

Die Wirkstoffe und die damit zubereiteten Mittel zeichnen sich im Gegensatz zu den besten hochwirksamen Kupferverbindungen durch eine sehr gute Pflanzenverträglichkeit aus, auch in empfindlichen Kulturen, wie Birnen und Äpfel.

Weiter besitzen die neuen Pflanzenschutzmittel den Vorteil, daß sie aus rein wäßriger Lösung angewendet werden können. Es treten daher bei ihrer Anwendung geringere Umweltbelastungen auf als bei der Behandlung mit bekannten Kupfer-Verbindungen.

In der Regel bilden die neuen Pflanzenschutzmittel nach dem Versprühen auf den überzogenen Gegenständen bzw. Pflanzen guthaftende Überzüge. Falls jedoch Schwierigkeiten hinsichtlich der Haftung auftreten, kann diese durch Zugabe eines Haftmittels (»sticker«) verbessert werden. Als Haftmittel eignet sich beispielsweise eine Styrol/n-Butylacrylat-Dispersion, die unter der Bezeichnung ®Acronal 567 D erhältlich ist.

Die neuen Mittel können in ihren Anwendungsformen auch zusammen mit anderen Wirkstoffen vorliegen, wie Herbiziden, Insektiziden, Wachstumsregulatoren oder Fungiziden.

Die folgenden Beispiele zeigen die biologische Wirkung.

## Beispiel A

Fungizide Wirksamkeit der Wirkstoffe gemäß Beispielen 1—7 gegen Phytophthora infestans an Tomaten.

Blätter von Tomatenpflanzen der Sorte »Professor Rudloff« werden mit wäßrigen Lösungen, die 0,024, 0,012, 0,006 und 0,003% Kupfer (bezogen auf metallisches Kupfer) enthalten, besprüht. Nach dem Antrocknen des Spritzbelags werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Der Versuch ergab, daß Lösungen mit einem Kupfergehalt von etwa 0,01% die Infektion sehr gut hemmen. Als besonders günstig haben sich die Fungizide der Beispiele 3, 4 und 5 erwiesen.

## Beispiel B

Bakterizide Wirkung gegen Erwinia amylovora an Birnen.

a) Birnenbäume der Sorte »Conference« wurden in einem Gebiet, in welchem die durch das Bakterium Erwinia amylovora verursachte Feuerbrandkrankheit stark auftritt, in einwöchigen Abständen mit 0,25%igen Lösungen des Wirkstoffes gemäß Beispiel 1 insgesamt zehnmal behandelt. Von 40 behandelten Bäumen wies nur einer eine Infektionsstelle auf; bei der gleichen Anzahl unbehandelter Bäume wurden 37 Infektionsstellen festgestellt.

b) In gleicher Weise wie unter a) beschrieben wurden Birnenbäume mit 0,25%igen Lösungen des Wirkstoffes gemäß Beispiel 2 gespritzt. Nur an 3 von 40 Bäumen wurden Infektionsstellen ermittelt.

Pflanzenschäden waren an den Bäumen nach der Behandlung nicht sichtbar.

## Beispiel C
## Pflanzenverträglichkeit

Apfelsämlinge der Sorte »Golden Delicious« wurden im 9-Blatt-Stadium mit wäßrigen Lösungen, die 0,04% Kupfer (bezogen auf metallisches Kupfer) enthielten, tropfnaß gespritzt. Die Versuchspflanzen wurden bei 18°C in einer Klimakammer mit Zusatzbeleuchtung aufgestellt. 14 Tage nach der Spritzung wurde das Ausmaß der Pflanzenschäden beurteilt.

In diesem Versuch zeigte das Präparat »Complex 200« in Konzentrationen Blattschäden (Nekrosen), bei denen z.B. die Substanz des Beispiels 2 einwandfrei vertragen wurde.

## Patentansprüche für die Vertragsstaaten: BE, CH, DE, FR, IT, LI, NL, SE

1. Pflanzenverträgliches Kupferamminsalz einer Polycarbonsäure, dadurch gekennzeichnet, daß es als Säurebestandteil 60—100% Acrylsäure und/oder Methacrylsäure und 0—40% Acrylsäure- oder Methacrylsäureester enthält.

2. Pestizid, enthaltend das pflanzenverträgliche Kupferamminsalz gemäß Anspruch 1.

3. Pflanzenfungizid, enthaltend das pflanzenverträgliche Kupferamminsalz gemäß Anspruch 1.

4. Pflanzenbakterizid, enthaltend das pflanzenverträgliche Kupferamminsalz gemäß Anspruch 1.

5. Verfahren zur Bekämpfung von Pflanzenschädlingen, dadurch gekennzeichnet, daß man die Pflanzen mit einem Pestizid gemäß Anspruch 2 behandelt.

6. Verfahren zur Bekämpfung von Pflanzenfungi, dadurch gekennzeichnet, daß man die Pflanzen mit einem Pflanzenfungizid gemäß Anspruch 3 behandelt.

7. Verfahren zur Bekämpfung von Pflanzenbakteriosen, dadurch gekennzeichnet, daß man die Pflanzen mit einem Pflanzenbakterizid gemäß Anspruch 4 behandelt.

8. Verfahren zum Überziehen von Pflanzen zwecks Schutz der Pflanzen bzw. Teilen davon vor Schädlingen, dadurch gekennzeichnet, daß man die Pflanzen oder deren Teile mit einer wäßrigen Lösung eines pflanzenverträglichen Kupfersalzes gemäß Anspruch 1 besprüht und die aufgesprühte Lösung trocknen läßt.

## Patentansprüche für den Vertragsstaat: AT

1. Pestizid, enthaltend ein pflanzenverträgliches Kupferamminsalz einer Polycarbonsäure, das als Säurebestandteil 60—100% Acrylsäure und/oder Methacrylsäure und 0—40% Acrylsäure- oder Methacrylsäureester enthält.

2. Pflanzenfungizid, enthaltend ein pflanzenverträgliches Kupferamminsalz gemäß Anspruch 1.

3. Pflanzenbakterizid, enthaltend ein pflanzenverträgliches Kupferamminsalz gemäß Anspruch 1.

4. Verfahren zur Bekämpfung von Pflanzenschädlingen, dadurch gekennzeichnet, daß man die Pflanzen mit einem Pestizid gemäß Anspruch 1 behandelt.

5. Verfahren zur Bekämpfung von Pflanzenfungi, dadurch gekennzeichnet, daß man die Pflanzen mit einem Pflanzenfungizid gemäß Anspruch 2 behandelt.

6. Verfahren zur Bekämpfung von Pflanzenbakteriosen, dadurch gekennzeichnet, daß man die Pflanzen mit einem Pflanzenbakterizid gemäß Anspruch 3 behandelt.

7. Verfahren zum Überziehen von Pflanzen zwecks Schutz der Pflanzen bzw. Teilen davon vor Schädlingen, dadurch gekennzeichnet, daß man die Pflanzen oder deren Teile mit einer wäßrigen Lösung eines pflanzenverträglichen Kupfersalzes gemäß Anspruch 1 besprüht und die aufgesprühte Lösung trocknen läßt.

## Claims for the Contracting states:
## BE, CH, H, DE, FR, IT, NL, SE

1. A plant-tolerated copper ammine salt of a polycarboxylic acid, wherein the acid is a copolymer of 60 to 100% of acrylic acid and/or methacrylic acid and 0 to 40% of an acrylic acid ester and/or methacrylic acid ester.

2. A pesticide containing a plant-tolerated copper ammine salt as claimed in claim 1.

3. A plant fungicide containing a plant-tolerated copper ammine salt as claimed in claim 1.

4. A plant bactericide containing a plant-tolerated copper ammine salt as claimed in claim 1.

5. A method of combating plant pest, wherein the plants are treated with a pesticide as claimed in claim 2.

6. A method of combating plant fungi, wherein the plants are treated with a plant fungicide as claimed in claim 3.

7. A method of combating plant bacterioses, wherein the plants are treated with a plant bactericide as claimed in claim 4.

8. A process for coating plants to protect them, or parts thereof, from pests, wherein the plants or parts thereof are sprayed with an aqueous solution of a plant-tolerated copper ammine salt as claimed in claim 1 and the sprayed-on solution is allowed to dry.

## Claims for the Contracting state: AT

1. A pesticide containing a plant-tolerated copper ammine salt of a polycarboxylic acid, wherein the acid is a copolymer of 60 to 100% of acrylic acid and/or methacrylic acid and 0 to 40% of an acrylic acid ester and/or methacrylic acid ester.

2. A plant fungicide containing a plant-tolerated copper ammine salt as defined in claim 1.

3. A plant bactericide containing a plant-tolerated copper ammine salt as defined in claim 1.

4. A method of combating plant pests, wherein the plants are treated with a pesticide as claimed in claim 1.

5. A method of combating plant fungi, wherein the plants are treated with a plant fungicide as claimed in claim 2.

6. A method of combating plant bacterioses, wherein the plants are treated with a plant bactericide as claimed in claim 3.

7. A process für coating plants to protect them, or parts thereof, from pests, wherein the plants or parts thereof are sprayed with an aqueous solution of a plant-tolerated copper ammine salt as defined in claim 1 and the sprayed-on solution is allowed to dry.

## Revendications pour les Etats constractants:
## BE, CH, H, DE, FR, IT, NL, SE

1. Sel cupramminique phytocompatible d'un acide polycarboxylique, caractérisé en ce qu'il comprend comme composante acide entre 60 et 100% d'acide acrylique et (ou) d'acide méthacrylique et entre 0 et 40% d'ester de l'acide acrylique ou de l'acide méthacrylique.

2. Pesticide, contenant un sel cupramminique phytocompatible selon la revendication 1.

3. Phytofongicide, contenant un sel cupramminique phytocompatible selon la revendication 1.

4. Phytobactéricide, contenant un sel cupramminique phytocompatible selon la revendication 1.

5. Procédé de lutte contre des parasites des plantes, caractérisé en ce que l'on traite les plantes avec un pesticide selon la revendication 2.

6. Procédé de lutte contre les champignons des plantes, caractérisé en ce que l'on traite les plantes avec un phytofongicide selon la revendication 3.

7. Procédé de lutte contre les phytobactérioses, caractérisé en ce que l'on traite les plantes avec un phytobactéricide selon la revendication 4.

8. Procédé pour l'enrobage de plantes pour protéger les plantes ou des parties de celle-ci des parasites, caractérisé en ce que l'on pulvérise sur les plantes ou des parties de celles-ci une solution aqueuse d'un sel de cuivre phytocompatible selon la revendication 1, puis on laisse sécher la solution pulvérisée.

## Revendications pour l'Etat constractant: AT

1. Pesticide, contenant un sel cupramminique phytocompatible d'un acide polycarboxylique comprenant comme fraction acide entre 60 et 100% d'acide acrylique et (ou) d'acide méthacrylique et entre 0 et 40% d'un ester de l'acide acrylique ou de l'acide méthacrylique.

2. Phytofongicide, contenant un sel cupramminique phytocompatible selon la revendication 1.

3. Phytobactéricide, contenant un sel cupramminique phytocompatible selon la revendication 1.

4. Procédé de lutte contre des parasites des plantes, caractérisé en ce que l'on traite les plantes avec un pesticide selon la revendication 1.

5. Procédé de lutte contre les champignons des plantes, caractérisé en ce que l'on traite les plantes avec un phytofongicide selon la revendication 2.

6. Procédé de lutte contre les phytobactérioses, caractérisé en ce que l'on traite les plantes avec un phytobactéricide selon la revendication 3.

7. Procédé pour l'enrobage de plantes pour protéger les plantes ou des parties de celles-ci

11  0 039 788

des parasites, caractérisé en ce que l'on pulvérise sur les plantes ou des parties de celles-ci une solution aqueuse d'un sel de cuivre phytocompatible selon la revendication 1, puis on laisse sécher la solution pulvérisée.